# EUROPEAN PATENT SPECIFICATION

(11) **EP 3 160 237 B1**
(45) Date of publication and mention of the grant of the patent: **18.09.2019**
(21) Application number: 15812628.4
(22) Date of filing: 22.06.2015
(51) Int. Cl.: A21D 15/08, A21D 13/00, A21D 8/04, A21D 13/24, A21D 13/28

(54) **METHOD OF MANUFACTURE OF PROBIOTIC FORTIFIED FOOD PRODUCTS**
VERFAHREN ZUR HERSTELLUNG PROBIOTISCH-ANGEREICHERTER LEBENSMITTELPRODUKTE
PROCÉDÉ DE FABRICATION DE PRODUITS ALIMENTAIRES ENRICHIS EN PROBIOTIQUES

(30) Priority: 25.06.2014 NZ 62669114
(43) Date of publication of application: 03.05.2017
(60) Divisional application: 19189227.2
(73) Proprietor: Goodman Fielder PTE. LTD, Singapore 068811 (SG)
(72) Inventor: NAG, Arup, Auckland 1010 (NZ); DAS, Shantanu, Auckland 1010 (NZ)
(74) Representative: Greaves Brewster LLP
(86) International application number: PCT/NZ2015/000044
(87) International publication number: WO 2015/199552

(56) References cited:
- US-A- 6 022 568
- US-A1- 2004 115 308
- US-A1- 2009 110 773
- US-A1- 2012 156 326
- SOUKOULIS, C. ET AL.: 'Probiotic edible films as a new strategy for developing functional bakery products: The case of pan bread' FOOD HYDROCOLLOIDS vol. 39, 30 January 2014, pages 231 - 242, XP055249459
- ALTAMIRANO-FORTOUL, R. ET AL.: 'Viability of some probiotic coatings in bread and its effect on the crust mechanical properties' FOOD HYDROCOLLOIDS vol. 29, 2012, pages 166 - 174, XP028422230
- ZYZELEQICZ, D. ET AL.: 'Probiotic Confectionery Products - Preparation and Properties' PROBIOTICS, INTECH, [Online] 2012, pages 261 - 306, XP055249462 ISBN: 978-953-51-0776-7 Retrieved from the Internet: <URL:http://www.intechopen.com/books/probio tics/probiotic-confectionery-products- preparation-and-properties>

## Description

### TECHNICAL FIELD

The present invention relates to probiotic fortified food products and methods of manufacturing same.

### BACKGROUND ART

Bread is a staple food in most part of the world. Therefore there has been significant interest in the food and health industries to use bread as a platform for functional and targeted health benefits, including probiotics.

Probiotics are viable microbial supplements that beneficially influence the recipient through its effects in the intestinal tract. Moreover, probiotics are one of the three food ingredients used to promote gut health (the others are non-digestible carbohydrates and bioactive plant metabolites).

To date, efforts to develop commercially useful functional bread containing the viable micro-organisms have not been developed and/or successfully marketed.

Primarily, this is because the focus over the past 5-10 years has been in developing bread that had probiotics integrally mixed within the bread prior to the baking process.

The main issue, as exemplified Zhang et al., 2014 (Journal of Food Engineering) is that most probiotic cultures that are safe to consume are also heat sensitive. Therefore the cell viability is significantly diminished during the baking process, which of course subsequently reduces the effectiveness of the probiotics in the gut when consumed. This is compared to live probiotic yogurts, for example, that have a therapeutically acceptable log CFU (colony forming unit, which is an estimate of the number of viable bacterial cells in the sample) of above 6.

Efforts have been attempted to address this problem.

For instance in Altamirano-Fortoul et al., 2012 (Food Hydrocolloids), the authors described micro-encapsulate probiotics and/or use starch as layers to protect the bacteria, prior to baking the bread. However, with this method of encapsulation, the bacterial cell viability decreased as a result of the heating. It would also be highly problematic to encapsulate bacteria for this purpose on a commercial level, and it is unlikely the same approach can be efficiently produced on a large scale or using high concentrations/amounts of probiotic to improve cell viability counts. As of yet, no commercially applicable encapsulation mechanisms have been developed to protect against high temperature baking.

Additionally, the author reported problematic changes in the physicochemical properties of the crust, increase in water activity and reduction in the failure force of the bread. The author suggested that the sensory evaluation provided a good acceptability of the bread. However, the present Applicant envisages that the added starch diminished the sensory perception profile (e.g. taste, feel and/or appearance) of the bread significantly.

In a similar approach, US Patent Application 20130115334 describes a granulated probiotic ingredient that was reported to withstand high temperature processing. When added to bread dough and baked at 200°C for 10 minutes, it was reported that 50-80% of the cell population survived.

US Patent Application 2010/0210000 attempted to solve the heating problem by using a heat stable, spore forming probiotic called *Bacillus coagulans* in the baking process.

The major drawback of using Bacillus is that it is a single species or strain, and is not as effective as a mix of other cultures like *Lactobacillus* and *bifido* micro-organisms which can target a range of consumers having different gut microbiota or ailments profile. It is established that probiotic efficacy varies quite substantially with the host digestive or immune system. Therefore, a very specific single bacterial strain such as that taught in US 2010/0210000 is bound to have very limited therapeutic benefits to the overall end users group.

Also, some species within *Bacillus* genre are pathogens (e.g., *Bacillus subtilis* causes food poisoning). The European Commission Health & Consumer Protection Directorate-General, (updated October 17, 2002) identified that some *Bacillus* strains *"may be problematic and should be accepted only for clearly defined strains, which have been tested negative for toxicity and pathogenicity in vitro and in vivo."* Therefore, it is thought that due to this potential hazard and/or public perception of a possible hazard, *Bacillus coagulans* enriched bread did not enter into mainstream. It is clear that bread enriched with most trusted probiotics (e.g. lactobacillus) does not exist at present, possibly due to lack of available technology solutions, such as encapsulation as discussed previously.

WO 1998009839 describes the use of a paste like formulation which is applied onto a food product, or within it as a filling for instance in a thin crispbread. The concept of using a paste was discussed as being advantageous as it mitigated probiotic growth, and at the same time, aided viability during the storage process. The probiotic was mixed with fat to provide the paste like consistency.

Similarly, EP1269857 and WO 2007/058614 describe similar filling or paste like compositions which is used for filling or covering a food product.

The main issue with these pastes is that they inherently will change the taste and appearance of the food product, which is often undesirable. In the context of bread, the commercial aim is to provide a probiotic containing bread that does not significantly change in taste or appearance compared to normal bread.

Secondly, the ingredients used to provide the paste-like consistency most typically will require added fats, non-natural thickeners or other excipients, and/or the need for sweeteners to mask the taste of the product. Again, this is disadvantageous, as it decreases the overall healthiness of the product, or at least can be perceived as such by consumers.

US 2009/0110773 describes a fruit snack with probiotics that is stable at room temperature, where the fruit snack centre is covered with a barrier layer which in turn is covered by an outer layer that contains heat sensitive ingredients such as probiotic cultures.

Soukoulis et al., 2014 (Food Hydrocolloids) discloses a composition to support viable probiotic films (with *Lactobacillus rhamnosus* GG) applied to the surface of breads. The composition includes hydrogels such as sodium alginate which are discussed as being important to retain good stability and microbial viability. Although the results suggest that the bread with the probiotic film shows no visual differences and stability compared to the control bread, it is not discussed whether the film had any effect on the sensory perception profile of the bread. Additionally, the manufacturing procedure for making the final product, as outlined in paragraphs 2.2 and 2.3 of Soukoulis *et al,* is inconvenient and time inefficient. Initial culture medias (PBS buffer and then MRS broth) are used to grow the culture and form aliquots to be frozen for subsequent use. Then in a subsequent step, the film forming solutions need to be prepared by re-dispersing dry materials (e.g. sodium alginate and other additives) in water, and then the *L. rhamnosus* frozen aliquots are added prior to applying to the bread.

US 2012/156326 describes a method for extending the mold-free shelf life and improving the flavour of baked goods by applying live yeast, including live probiotic yeast (*Saccharomyces cerevisiae* var. *boulardii*), on the surface of the baked goods.

WO 2002/065840 describes a composition which is applied on cereals, directly after fermentation. The process is considered advantageous because it does not require high temperature drying. The cereal was discussed to display good storage stability, good retention of a high CFU and similar appearance to the non-probiotic cereals.

Yet it is important to consider these final cereal products in WO 2002/065840 have a water activity of 0.2, such that the cereal was able to absorb the residual moisture coming in with the probiotics (no drying step involved) and overall, the water activity of the final mix did not go up above 0.2. Bread products, on the other hand, has a much higher water activity of at least 0.5 and most often about 0.8 to 0.9. For instance, water activities bread crumbs are 0.8. to 0.9¹, bread crusts are about 0.7 to 0.8² and cereal bars are about 0.6 to 0.7³.
(1): Curti et al, The use of potato fibre to improve bread physicochemical properties during storage, Food Chemistry 2016; 195: 64-70. (2) Curti et al, Effect of the addition of bran fractions on bread properties, Journal of Cereal Science 2013; (3) Aigester et al, Physicochemical properties and sensory attributes of resistant starch-supplemented granola bars and cereals. Food Science and Technology 2011.

Therefore the challenge in offering probiotic stability under ambient storage and higher water activities above about 0.5, an in particular bread products, is much more difficult. The teachings of WO 2002/065840 do not provide any useful information towards higher water activity products such as bread.

Additionally, the composition in WO 2002/065840 includes a wide number of excipients including anti-foaming agent, yeast extract, meat peptone, and buffer salts (amongst others).

In US2004/0115308 it is disclosed to apply fresh biomass to a consumable product. The biomass is obtained by growing probiotics is a medium that is not exclusively milk-based.

Therefore, it would be beneficial to identify a product (and related process) that:
- allows a therapeutically and commercially acceptable live probiotic count (log colony forming units, CFU) for the resulting food product such as bread which has a high water activity, particularly above 0.5;
- provides a very similar or substantially identical taste and/or appearance of the normal (non-probiotic) food product;
- is storage stable;
- does not require additional synthetic excipients or thickeners;
- is simple and efficient to make, and requires only basic food processing machinery;
- is adaptable to a wide range of food products besides baked goods or bread; and/or
- is adaptable to be used with a wide range or combinations of probiotic strains.

More broadly speaking, it is an object of the present invention to address the foregoing problems or at least to provide the public with a useful choice.

Further aspects and advantages of the present invention will become apparent from the ensuing description which is given by way of example only.

### DISCLOSURE OF THE INVENTION

According to a first aspect of the present invention there is provided a method of manufacturing a probiotic fortified food product, optionally bread, bread snack or muesli bar, including
a. pre-inoculating a composition that comprises an amount of milk, milk solids, or reconstituted milk with an amount of the single species of probiotic micro-organism or combination of different probiotic micro-organisms, wherein other than the probiotic micro-organism, the composition only includes components which are either fully milk-derived or are inherently found in milk;
b. incubating the composition during an early stationary growth phase of the probiotic micro-organism(s) until a probiotic culture in the composition is achieved with a cell count above 7 log CFU ml⁻¹;
c. applying the composition including the at least one probiotic organism to at least one portion of a surface of the food product.

Also described herein is a probiotic fortified food product
characterised in that at least a portion of a surface of the food product includes a composition including at least one probiotic microorganism, wherein the composition includes a base which is fully milk-derived and/or includes components inherently found in milk.

Also described herein is use of a fully milk derived composition
for the manufacture of a probiotic fortified food product as described herein,
for the improvement, treatment or prevention of an intestinal tract dysfunction or disorder, and/or conditions relating to same.

A use of a fully milk derived composition
for the manufacture of a probiotic fortified food product as described herein
for the improvement of immunity, action as an anti allergenic, treat eczema, and/or aiding in cholesterol lowering.

Further described is a method of treatment, improvement or prevention of an intestinal tract dysfunction or disorder, and/or conditions relating to same by administering a probiotic fortified food product as described herein to person or other animal in need thereof.

Also described is a method of treatment by administering a probiotic fortified food product as described herein to improve immunity, act as an anti allergenic, treat eczema, and/or aid in cholesterol lowering to person or other animal in need thereof.

A key advantage of the present invention is the ability to prepare a probiotic fortified food product such as bread with a water activity level above about 0.5, with a probiotic micro-organism at commercially and therapeutically viable cell counts (over 7.0 log CFU per serving).

The present invention is also easy to prepare, and has no adverse impact on the overall sensory perception on the food product's taste, colour or visual appearance.

Advantageously the present invention relies on only a milk-derived composition.

Further advantages and preferred features of the invention are discussed below.

### DEFINITIONS AND PREFERRED EMBODIMENTS

Throughout this specification the term base (when referring to the base of the composition) should be taken as meaning all the components, or matter present in the composition other than the probiotic micro-organism(s), and if necessary a liquid such as water which may be used to reconstitute a powder form of the composition into a liquid.

Throughout this specification the term milk should be taken as meaning a liquid produced from the mammary glands of a mammal. The milk derived features according to the present invention may be sourced from any number of mammals; however, the most commercially applicable mammalian milk includes cow, goat, sheep, deer, camel, buffalo and so forth.

Throughout this specification the term food product should be taken as meaning any edible substance which is consumed to provide nutritional support for the body.

Throughout the remainder of this specification the food product will be referred to, for simplicity, as baked bread. However, it should be appreciated that the inventive concept described herein may similarly be applied to other types of food products (either baked or unbaked), for example muesli bars, breakfast cereals, biscuits, muffins, pizza bases, candy bars and so forth, particularly those with a higher water activity above 0.5. The present invention has particular application to farinaceous products. The Applicant also envisages the inventive concept may have particular commercial application to those partaking in the low FODMAP diet⁴, which is
⁴ FODMAP stands for "Fermentable Oligosaccharides (fructans and galacto-oligosaccharides) Disaccharides (lactose) Monosaccharides (fructose) and Polyols (sorbitol, mannitol, xylitol and maltitol). thought to significantly relieve irritable bowel syndrome (IBS). However, a disadvantage of the FODMAP diet is that it is often low in fibre and prebiotics. This may negatively affect the gut microflora. Instead, a probiotic fortified low FODMAP bread as per the present invention may be particularly useful to use in the low FODMAP diet as it may also provide the probiotics to maintain healthy gut microflora.
⁴ FODMAP stands for "Fermentable Oligosaccharides (fructans and galacto-oligosaccharides) Disaccharides (lactose) Monosaccharides (fructose) and Polyols (sorbitol, mannitol, xylitol and maltitol).

Throughout this specification the term probiotic fortified food product should be taken as meaning any food product such as a bread that has been adapted according to the present invention to include a probiotic composition on a portion of its surface.

Throughout this specification the term surface should be taken as meaning the external portion of the bread, also known as the crust which is formed as a result of baking. The crust forms around the entire surface of the bread, including bottom, sides, and top of the baked bread. Throughout this specification, the term thin layer should be taken as meaning a coating, membrane, film or skin that is applied (for instance via a spray or via spreading or smearing of the composition) to at least one portion on the surface of the bread.

Throughout this specification, the term "substantially dried (or dried) should be taken as meaning the thin layer has had the majority, substantially, or all the moisture removed from the thin layer after its application to the surface of the bread.

It should therefore be appreciated that the term dry, dried, or substantially dried should allow for some moisture to still be retained in the thin layer, but essentially the moisture not be freely available. The term dry or dried in relation to the thin layer should also be considered to result in a probiotic fortified food product that has similar moisture content and/or water activity (a_{w}) compared to the unmodified food product.

Water activity is defined as a ratio, namely the partial vapour pressure of water in a substance divided by the standard state partial vapour pressure of water. In food science, the standard state is typically defined as the partial vapour pressure of pure water at the same temperature. Pure water has a water activity of one.

Throughout this specification, and as defined by the Food and Agricultural Organization of the United Nation and WHO, the term probiotic should be taken as meaning a collection of live micro-organisms (bacteria and/or yeast) which, when administered orally in adequate amounts, are able to confer a health benefit on the host. The collection should be taken as encompassing either a single species of micro-organism or combination of species of micro-organisms.

Generally, a particular micro-organism may be considered a probiotic when it is able to satisfy a number of different criteria. These include reasonable survival through physiological and manufacturing demands, as well as the ability to exert beneficial effects on the host (B. O'Grady, 2005).

A large number of species are now known as being probiotic. These include the *Lactobacilli* and *Bifidobacterium* - these are of high commercial importance. Consequently, they have been used widely in the food industry as probiotic organisms. *L. acidophilus, L. casei* strain *Shirota, L. rhamnosus* and *L. reuteri* are popular choices and have a long application history followed by some *Bifidobacterium* species, and also a few non-lactics which are mainly used in pharmaceutical applications (Holzapfel *et. al.,* 1998). Probiotics are commercially marketed either in a lyophilized form or as fermented food products.

Scientific literature has conventionally focused on the health benefits from either one or a combination of different strains of probiotic micro-organisms towards immune modulation and improving the strength of the gut mucosal barrier. These effects are achieved by modification of gut microflora, capability to adhere with intestinal mucosa which in turn helps to prevent pathogen adherence or activation, by modifying the dietary proteins and bacterial enzyme capacity and by influencing the gut mucosal permeability (Holzapfel *et. al.,* 1998).

In 2000, a review by Kailasapathy and Chin found that probiotics have been reported to also play a beneficial role in immunity, lowering cholesterol, improving lactose tolerance and even preventing some cancers. Also, probiotics have been associated with digestive and respiratory functions, and prevention of infectious diseases in children and other high risk populations (sourced from WHO, 2001). On this basis, the present invention is considered to also have a therapeutic benefit to treating any one or combination of a wide range of conditions and/or simply improving overall health as described in such literature.

Throughout this specification the term inoculate, inoculated, and inoculation should be taken as meaning the process of adding at least one bacterial strain to the composition, and then most typically propagation of the probiotic bacterial strain(s) via a fermentation process to a commercially and therapeutically level of above 7.0 log CFU ml⁻¹, and more ideally above 9.0 log CFU ml⁻¹. It should be appreciated that the pre-fermentation process to achieve the 7.0 log CFU ml-1 does not need to be performed as part of the invention, and may alternatively be supplied by a third party and then added to the composition at sufficient amounts/ concentrations to provide a therapeutic effect according to the present invention. However, for reasons outlined in the specification, it is preferred to conduct the pre-fermentation step inhouse as it allows effective control of the bacterial growth phase, is convenient, and results show improved cell viability over time - especially after application to the bread's surface. For instance, the pre-fermentation step of about 16 hours may be conducted overnight, and in the following morning, the fermented composition may then be freshly applied to the bread.

### Method of preparing the probiotic fortified food product

The method of preparing a probiotic fortified food product is characterised by:
a) applying a composition inoculated with at least one probiotic organism to at least one portion of the surface of the food product, wherein the composition includes a base which is fully milk derived and/or includes components inherently found in milk.

Preferably, step a) is performed after cooking the food product. However, it should be realised that the present invention need not be limited to cooked or baked food products, and therefore could be applicable to non-cooked or non-baked products.

A key advantageous difference in the present method compared to the prior art methods is that only a moderate drying step is required after the composition is applied as a thin layer to the food product, as will be discussed below.

In the case of bread, unlike the prior art, the probiotic is not intrinsically applied into the bread and then baked, which has numerous disadvantages including a loss of probiotic cell viability. Therefore, the invention allows one to move away from the need to use heat stable probiotics used during the baking process, and/or altering the baking conditions needed to achieve optimal baked bread.

Compared to the prior art paste approaches, the advantageous step of the invention is achievable because of the discovery that when the composition is applied as a thin layer, it is able to be quickly dried, adhere to the bread's surface and unexpectedly still retain an achievable cell viability.

Cell viability continues even after considerable storage and when retaining much higher water activity level than prior art cereal based probiotic compositions. There is also very little water activity change compared to that seen in normal, non-probiotic bread, which depending on the type of bread typically ranges from an a_{w} value between 0.5 to 0.9. As noted previously, most bread crumbs / crusts have a water activity in the range between 0.7 to 0.9. Trial samples tested by the Applicant on buns / ciabatta confirmed water activities of about 0.7 to 0.8.

Furthermore, unlike the pastes seen in the prior art, the resulting food product retains substantially the same taste and look to the non-probiotic breads as illustrated in the examples. This is simply not possible with pastes.

Finally, the present method is very efficient and requires only standard baking and kitchen facilities.

Preferably, step a) includes applying between 200 µl to 2 ml of probiotic culture to the bread.

It should be appreciated that the amount applied to the bread is largely dependent on the size of the bread. One skilled in the art would appreciate the intention is to provide a therapeutically effective amount or dosage of viable cells per serving, namely above 7.0 log CFU per serving. A typical bread serving size would be about 10 to 100 grams, and more likely 30 to 50 grams. Therefore for a single serve bread, the amount applied is generally less than a larger loaf. For a larger loaf, it may become more important to ensure the composition is spread evenly over a surface (for instance the top surface) to ensure that when a piece of bread is cut off, it may provide an adequate amount of the probiotic on each serving.

More preferably, step a) includes applying between 500 µl to 1 ml of probiotic culture to the bread.

In preliminary studies, the inventors discovered that these amounts were particularly advantageous to avoid unnecessary increases in moisture content of the bread which needs to be dried off to result in the bread being close to its original moisture content without compromising the bread texture and other organoleptic properties.

Also, longer/harsher drying conditions would be needed if greater amounts of composition are added (or if the composition is a higher viscosity like a paste). Harsher drying may also lead to a greater loss of probiotic viability.

Coupled with these technical hurdles, the inventors also needed to ensure an appropriate quantity of probiotic is present in the thin layer on the surface of the bread (preferably at least across the whole top surface of the bread), and still provide suitable cell viability and dosages.

Preferably, step a) includes ensuring the thin layer is less than 0.1 mm thick.

More preferably, step a) includes ensuring the thin layer is less than 0.05 mm thick.

As discussed above, the inventors identified that having a thin layer of less than 0.1 mm, or more preferably less than 0.05 mm is advantageous to improve the efficiency of the drying process, avoiding water moisture issues whilst also avoiding losses in viable cell counts, and also helping to ensure the composition adheres to the surface of the bread, and helping to avoid changes to the taste and appearance of the probiotic bread.

Preferably, step a) includes spreading the thin layer across an entire side surface of the food product.

For example, the side surface may be the top portion of a baked bread. In other instances, it may be applicable to apply the thin layer across both the top portion together with a number of other surfaces (e.g. elongate sides, end portions, and bottom) of the baked bread. In another example of an oval shaped roll, the thin layer could be applied to the entire roll's outer surface. This may be useful in an instance when a greater probiotic dosage is required, or if the probiotic has a shorter shelf life.

Preferably, step a) includes spraying the composition on the surface of the food product.

This is advantageous as it allows quick application, and also helps to provide the thin layer as a result of the spraying process. Automation may also be useful to allow even consistency across the surface of the bread.

As one alternative, step a) may be conducted using a dispenser (e.g. pipette) and then the composition may be spread as a thin layer using a spatula or brush.

Preferably, the method also includes drying the composition after it is applied to the surface of the food product.

In order to improve the results, the conditions of the drying should take into account a number of factors, including trying to avoid impact on the product sensory effects, optimal survival of the bacteria in the drying process, as well as improved survival of the bacteria.

Preferably, the method includes a further step b), which includes drying the bread until the thin layer includes a water activity (a_{w}) level between 0.5 to 0.9. This range is chosen because it represents the typical water activity of bread type products. The aim is to match the water activity of the composition to the water activity of the bread, so as to help avoid any change in sensory perception of the bread.

As discussed previously, one of the significant advantages and distinguishing features of the invention is the ability to retain stability of the composition and probiotic viability at a higher water activity levels of the food product, for instance at about 0.7 to 0.9 in the case of bread. This is very different to the focus of WO 2002/065840 which has a much different composition base and is applied to cereals with a very low water activity of about 0.2. However, it should be appreciated that the composition may be applicable to food products with a water activity level below 0.5. However, the ability to work with food products with a higher water activity level above 0.5 is considered to be particularly beneficial and commercially important.

Preferably, step b) includes drying the composition until the thin layer of composition includes a water activity (a_{w}) level between about 0.5 to 0.8.

As discussed previously, the preferred aim is to closely match the original water activity (a_{w}) of the bread before applying the thin layer of the composition.

Preferably, step b) includes drying the thin layer at a temperature below 70°C.

A higher drying temperature beyond 70°C may not only destroy the live probiotic cells, but may also change the bread texture into being more crispy with a crumbling crust.

More preferably, step b) includes drying the thin layer at a temperature between 30°C to 70°C.

It is possible that the drying step may be performed below 30°C, but preliminary trials suggest it could negatively affect product quality, and unnecessarily increase the drying time required.

Most preferably, step b) includes drying the thin layer at a temperature between 50°C to 60°C.

These preferred drying temperatures are very different to temperatures used to bake bread, typically at about 180°C.

Preferably, step b) is performed for between 10 to 30 minutes.

The drying time may be dependent on numerous factors including air temperature, air flow, humidity, the method of composition application, the shape of the bread, and so forth. It should be appreciated that the conditions and the drying time may be modified significantly, but the intention is to return the water activity of the bread to near the original water activity before the composition was applied. Also, the drying step should be sufficient to ensure the composition effectively sticks to the bread. A drying time between 10-30 minutes, and particularly 15 to 20 minutes, at a temperature between 50°C to 60°C was found to be ideal for operational efficiency and product quality.

Most preferably, step b) is performed for between 15 to 20 minutes.

The inventors consider that a suitable and convenient machine to perform drying step b) is a convention type forced air.

Other options to perform step b) include using a continuous conveyor belt passing through a hot air tunnel. It is possible the drying step may be performed simply by passive evaporation, for instance under room temperature conditions for about 24 hours. However, the disadvantages may include that the micro-organisms may continue to grow during this time, leading to possible loss of cell viability and or adverse changes to taste of the food product.

After step b) the food product may then be stored under normal bread conditions (e.g. up to five days at 25°C without any substantial loss of cell viability of the probiotic culture. This is illustrated in the Examples. It is also possible to refrigerate or freeze the resulting probiotic fortified food product. In such cases, the shelf life may be extended.

The inventors foresee that the present invention is particularly applicable and advantageous towards the fortification of probiotic fortified food products. However, it should be understood that the invention may also be utilised in a similar way for fortification of food products with other functional ingredients, such as vitamins applied to the surface of bread. For example, some vitamins such as vitamin C are heat sensitive.

Also, the present invention may be useful for a combination of two or more different functional ingredients, such as a probiotic and a vitamin.

Clearly the invention is particularly attractive to address problems wherein the functional ingredient(s) are heat sensitive, like probiotics.

### Preferred features of the composition used in the method of manufacture

Preferably, the composition has a viable cell count above 7.0 log CFU ml⁻¹.

It should be appreciated that the composition should ideally be applied in an amount such that a single serving includes at least 7.0 log CFU, and more preferably at least 9.0 log CFU. This reflects the FAO/WHO (2003). Standard for Fermented Milks. CODEX STAN 243, which provides guidance that for any food products which include probiotic cultures, the product needs to contain a cell viability of at least 10⁶ CFU per g to confer the desired health benefits.

More preferably, the composition has a viable cell count above 9.0 log CFU ml⁻¹.

The advantage of having higher viable cell count of above 9.0 log CFU ml⁻¹ in the probiotic culture is that it allows for slight decreases in viable cells as a result of the drying conditions and/or storage of the probiotic fortified food product, and still maintain above the acceptable viable cell count of 7.0 log CFU ml⁻¹. However, as discussed herein and as shown in the Examples, the inventors were very surprised to see how well the probiotic on the food product remained stable, with very minimal losses in cell viability.

Preferably, the probiotic micro-organism(s) in the composition is selected from the group consisting of a lactic acid bacteria, non-lactic acid bacteria and non-pathogenic yeast or combination thereof. Essentially, any probiotic micro-organism may be used that is safe to consume and displays (or is thought to display) therapeutic probiotic effectiveness in the gut. Micro-organisms that have good shelf life are preferred.

More preferably, the probiotic micro-organism in the composition is selected from the group consisting of *Lactobacillus acidophilus, Lactobacillus bulgaricus, Lactobacillus casei, Lactobacillus bifidus* (bifidobacterium), *Lactobactillus plantarum, Streptococcus thermophilus,* and combinations thereof.

These micro-organisms are used regularly in the food industry and therefore are commercially acceptable from the perspective of the manufacturer and consumer. Yakult™ is one example of a probiotic product using the bacterium *Lactobacillus casei* Shirota. This represents a product that has a singular probiotic strain. Activate™ is another example of a unique singular bifibobacterium, or commercially known as Bifio-Defenis™, with proven health benefits. *Lactobacillus plantarum* is a probiotic micro-organism that has been well linked to therapeutic effectiveness towards intestinal health of people with irritable bowel syndrome, which is particularly prevalent in the Western world.

The composition may include just a single probiotic strain, or alternatively a combination of probiotic strains.

The commercial advantage of using a single strain may be claiming the final product as a probiotic fortified bread. Yet, a combination of probiotic strains may be useful to provide a broader or synergistic therapeutic effect to the gut.

The composition includes a milk-derived base. The base is made up of a number of excipients which are either milk-derived or are those which may be added to the base, but which inherently are found in milk.

Advantageously, the base of the composition is able to
a) act as a growth media to ferment the microbes, if required, to above a preferred CFU level and beneficially just at the end of the initial growth phase;
b) offer a beneficially low viscosity to allow easy spreading and application as a thin layer to the surface of the food product;
c) allow efficient and effective adherence to the surface of the food product and;
d) retain viable counts of probiotics for a commercially useful period after application to the food product. For example, in preliminary trials on bread, the composition retained optimal CFU over a period of five days storage at 25°C (standard trial period for bread storage stability). when applying Yukult® and Activate® to the surface of bread, preliminary results were poorer (faster decline of cell viability) compared to the trial group where the composition was pre-fermented just prior to application onto the bread.

The unexpected advantages of using milk-derived component(s) as the composition's base will be discussed in greater depth within this specification. However, one over-riding commercial focus is to achieve the desirable CFU required for probiotics, stability, efficient and effective application to the food product and retain beneficial sensory perception, but without having to resort on any non-milk derived ingredients as is seen heavily in the prior art - for example in WO 2002/065840.

Preferably, the base of the composition includes an amount of milk, milk solid(s), or reconstituted milk (preferably skimmed milk).

It was unexpectedly found that these components from milk are sufficient to provide the composition's base to allow the inventive method to be performed with beneficial results.

For example, the composition may include about 12% reconstituted skimmed milk.

Preferably, the base of the composition has less than 5% fat.

More preferably, the base of the composition has substantially no fat.

For these compositions, fat was not required. This is very different to the prior art techniques describing pastes, which use high fat content in the paste to increase the viscosity and prevent growth and retain the micro-organisms.

Preferably, the base in the composition includes a coagulant. In this case, the coagulant may be lactic acid which is developed by fermentation in the media.

The inventors also surprisingly discovered that the coagulated milk, milk proteins, or other milk component provides a significant advantage in helping the composition stick to the surface of the food product.

Also, the coagulation beneficially did not overly affect the fermentation process of the micro-organism (if this is required as an initial step to boost the cell count), nor does it overly affect the viscosity and/or spreadability of the composition.

A wide variety of components may be used to achieve this at least partial coagulation effect, and those skilled in the art would appreciate this.

Preferably, the component configured to at least partially coagulate the milk is lactic acid. An advantage of using lactic acid is that it is a milk-derivable compound. This is in line with the advantage seen by using primarily milk-based components to achieve the results of the invention, without having to resort to synthetic or extrinsically provided (i.e. non-milk based) excipients. In other words, it retains the commercially beneficial effect of being made using natural ingredients, and is in general alignment with providing a healthy food product.

Preferably, the base of the composition includes at least one type of sugar.

The presence of a sugar was surprisingly found to be very useful to achieve numerous beneficial effects, as noted below:
- significantly aids the fermentation process to increase the probiotic count when the composition is being used for the pre-fermentation process;
- helps the composition adhere to the surface of the bread; and/or
- helps retain good probiotic viability of the composition before and after application to the surface of the bread.

Preferably, the sugar is inherently provided from a milk source.

This is in line with an over-riding commercial focus of the Applicant to maintain the composition to be fully milk-derived or only including components in the base which inherently are found in milk.

Preferably, the sugar is selected from lactose, glucose, and/or galactose.

Advantageously, milk includes these types of sugars. Lactose accounts for about 40% of a cow's milk calorie count, and about 5% w/v of the milk's contents. Lactose is a disaccharide made of two simple sugars - glucose and galactose, and therefore either one or both of such primary sugars may be also be used in the present invention. With the self-imposed commercial aim of working with milk-derivable sugars, the Applicant was surprisingly able to provide a highly functioning composition which allows pre-fermentation, effective adhering properties, and excellent probiotic viability retention.

It should be appreciated that the base of the composition may be supplemented with an additional amount of sugar. Although technically, any food grade sugar may be used to supplement the composition, any supplemented sugar should be milk-derived, or at least inherently found in milk, to remain aligned with the commercial focus of the Applicant. For instance, food grade sugars naturally found, or obtainable, in milk (ie such as lactose, glucose or galactose) may be used. It is not necessary for these added sugars to be isolated, extracted or obtained from milk. For instance, glucose obtained from vegetable sources may be used.

Most preferably the sugar is lactose.

The inventors identified in preliminary trials that a composition including 8% reconstituted skim milk supplemented with 4% milk derived lactose or glucose retained above 9.0 log CFU ml⁻¹ after 24 hours storage (and following application to bread when the starting cell count was about 9.25 log CFU per serve). Surprisingly, lactose performed slightly better than glucose at retaining cell viability. This was compared to a composition with 12% reconstituted skim milk (no supplemented sugar) which saw a more substantial decrease to 8.7 log CFU ml⁻¹ after 24 hours storage. The fact that lactose represents the most readily available milk-based sugar means it may be most commercially viable option and aligns with the commercial focus of the product and methodology. In the case of lactose-free or lactose reduced based compositions, one could substitute lactose for glucose.

Preferably, the base of the composition includes between 2% to 20% w/v total sugar, or more preferably 3 to 10% w/v total sugar.

Reconstituted skim milk naturally includes about 3% w/v sugar (in a situation where a 10 fold dilution of skimmed milk powder). The Applicant has identified that although reconstituted skim milk alone (which primarily includes lactose inherently) achieves beneficial results, further supplementation of the composition to include an additional amount of sugar (for instance 4-10% w/v added sugar) improves the results as exemplified with respect to the cell viability counts.

Therefore in one embodiment the composition is reconstituted milk solids and enriched with lactose. This composition was found to be particularly effective in achieving the desirable results, and in particular cell viability. Beneficially, the composition is fully milk derived.

Preferably, the composition has a viscosity below 4 cP (centipoise).

The preferred viscosity helps the composition to be applied as a thin layer to the surface of the bread. For instance, as a low viscosity, the composition may be applied via a spray, or alternately pipetted and then spread easily with a spreader, such as a spatula.

Preferably, the composition has a viscosity between 1 and 3 cP.

This preferred viscosity of the resulting composition is similar to milk, as this helps to allow the composition to be easily spread and dried on the food product, as further elaborated on below.

The preferred viscosity helps to further differentiate over pastes which clearly represent a different approach to the present invention. The paste-like compositions of the prior art relied on high viscosity to control cell growth and viability of the toppings/fillings used for food products. This does not address a key object of the present invention in terms of providing a probiotic fortified bread product that does not substantially differ in terms of taste and appearance from the normal product. The paste-like toppings/fillings teach away from the present invention.

Also, this helps the thin layer on the bread to be dried more efficiently, thereby reducing the moisture content to bring it back to the level of original bread before the thin layer is applied. Since it is a standard industrial practice to maintain the moisture content and/or water activity of any bread at a level which keeps it stable for at least five days at ambient temperature, this invented process also aims to maintain the post drying moisture content as close as possible to the original bread. Unexpectedly, the inventors found the cell viability was able to be kept at a commercially and therapeutically acceptable level after moderate drying processes. Also, unexpectedly, the inventors found the cell viability was able to be kept at a commercially and therapeutically acceptable level after moderate drying processes.

Furthermore, the ability to apply the composition as a thin layer (aided by the low viscosity) helps the composition be substantially transparent and/or non-visible on the bread once dried. This also helps to avoid adversely affecting taste, feel or look of the probiotic fortified bread compared to the normal bread.

### Pre-fermentation

It should be appreciated that the probiotic micro-organisms may be pre-fermented in the composition, or may be added at a suitable level in order to achieve a CFU above 7.0 log ml⁻¹, or more preferably above 9.0 log ml⁻¹.

Hence, the use of the term "pre-fermented" in such as case may have been conducted in a separate step, for instance by a third party supplying the concentrated bacteria sample, or by the manufacturer of the food product, just as a separate step.

Regardless, the end result of the composition should be that the amount and concentration of the bacteria added to the composition is sufficient enough to provide the appropriate therapeutic probiotic level (i.e. above 7.0 log CFU ml⁻¹).

For example, freeze dried, frozen or other form of concentrated bacteria as a source of live cells may be added to carrying material.

Alternatively, the method of preparing the probiotic culture composition includes pre-fermenting the micro-organism in the base of the composition to achieve a desired cell count above 7.0 log CFU ml⁻¹.

As noted previously, an advantage of the composition is that the base of the composition may also act as the growth medium to allow the fermentation process of the micro-organism. Therefore, as a matter of manufacturing convenience, there is no need to adapt the composition from a fermentation step into a new composition for application to the bread's surface.

The base of the composition typically will be sterilised before use for safety and control purposes. For example, the sterilisation may include heating at about 90°C for about 15 minutes.

Preferably, the inoculant (or starter) of probiotic micro-organism is added to the base of the composition after it has cooled substantially from the sterilisation temperature. The inventors consider a 0.05% w/w inoculation may be appropriate.

Preferably, the method includes incubating the base of the composition during the early stationary growth phase.

For example, in the case of *Lactobacillus casei* 431 or LC431, the incubation may be performed for 16 hours at 37°C to obtain suitable cell counts.

Preferably, the method includes incubating the base of the composition until the probiotic culture has a viable cell count above 7 log CFU ml⁻¹.

More preferably, the method includes incubating the base of the composition until the probiotic culture has a viable cell count above 9.0 log CFU ml⁻¹.

The advantages of these viable cell counts are discussed previously.

Preferably, the method includes homogenizing or stirring the resulting medium (typically a curd) to produce a spreadable or sprayable composition.

The pH of the resulting curd medium was recorded as 4.45 after a 16 hour trial incubation using *Lactobacillus casei* 431. The pH did not negatively affect the resulting cell viability of the probiotic.

Summary of the advantages of the present invention may include, but should not be limited to, one or more of the following:
- the simplicity of the composition's base is being milk-derived is beneficial given it is easy to source, manufacture and use, and will have good public acceptance;
- the base, being milk-derived, allows pre-fermentation of the microbes, helps to keep the viscosity of the composition to within the preferred range to help with application and/or spreadability on to the bread's surface, help it to adhere to the surface of the bread, and helps to retain good viability of the probiotics for a commercially useful period of time;
- able to retain optimal probiotic viability (CFU counts) at high water activity levels of food products such as bread;
- ability to prepare a food product such as bread fortified with a probiotic at commercially and therapeutically viable cell counts (over 7.0 log CFU ml⁻¹);
- good stability of viable probiotic cells after standard five day trial at 25°C (normal shelf life of bread);
- avoids use of disadvantageous pastes (which affect taste and appearance) or the need to integrally apply to the bread before baking (which ultimately reduces cell viability significantly);
- the application as a thin layer allows for very effective drying, which is important to the resulting product does not have a significantly altered moisture content and/or water activity;
- no specialised equipment or high temperature resistant probiotic strains required;
- no substantial adverse impact on the overall sensory perception of the probiotic fortified bread compared to normal control bread (taste, feel or appearance); and/or
- no impact on storage stability of bread.

### BRIEF DESCRIPTION OF THE DRAWINGS

Further aspects of the present invention will become apparent from the ensuing description which is given by way of example only and with reference to the accompanying drawings in which:
- Figure 1: Analysis of Log CFU following preparation and storage of fortified LC431 probiotic bread; and
- Figure 2: Analysis of Log CFU following preparation and storage of fortified LC431, BB12 and LA05 combination probiotic bread.
- Figure 3: Analysis of Log CFU following preparation and storage of a fortified LC431 composition compared to commercially available Yakult and Activate probiotic drinks at various stages of manufacture of probiotic bread.
- Figure 4: Analysis of Log CFU following preparation and storage of fortified *Lactobacillus plantarum* 299V.

### BEST MODES FOR CARRYING OUT THE INVENTION

### Example 1: Preparation of the growth media

A growth media for *Lactobacillus casei* LC431 was made according to the following steps.
1. Prepare a media including 8.0% w/w reconstituted skim milk and 4.0% w/w glucose or lactose.
2. Heat the media to 90°C for 15 minutes.
3. Cool to room temperature.

### Example 2: Preparation of the pre-fermented priobiotic composition

The *Lactobacillus casei* LC431 probiotic composition was prepared as follows:
1. Add 0.05% w/w inoculant of freeze dried LC431 culture to the growth media as pre-prepared in Example 1.
2. Incubate the inoculated growth media for 16 hours at 37°C.
3. Homogenize the resulting soft curd to form a spreadable/sprayable liquid.

### Example 3: Preparation of the probiotic fortified bread

A fortified *Lactobacillus casei* LC431 probiotic bread was prepared as follows:
a) Spray 500 µl to 1 ml of composition (see Example 2) to a surface of a pre-baked bread loaf, ensuring a thin layer is evenly applied to the surface
b) Dry the thin layer onto the bread's surface in a convention type forced air oven for 15 minutes at 50°C.
c) Store the probiotic fortified bread at 25°C or lower.

### Example 4: Analysis of Log CFU following preparation and storage of fortified L. casei 431 (LC431) probiotic bread

A probiotic fortified bread was prepared according to Example 3. The inventors tested the viability of the LC431 cells (Log CFU) in three different trials. Log CFU was recorded for the control LC431, after application/drying, as well as following storage for five days at 25°C (standard storage testing conditions for bread).

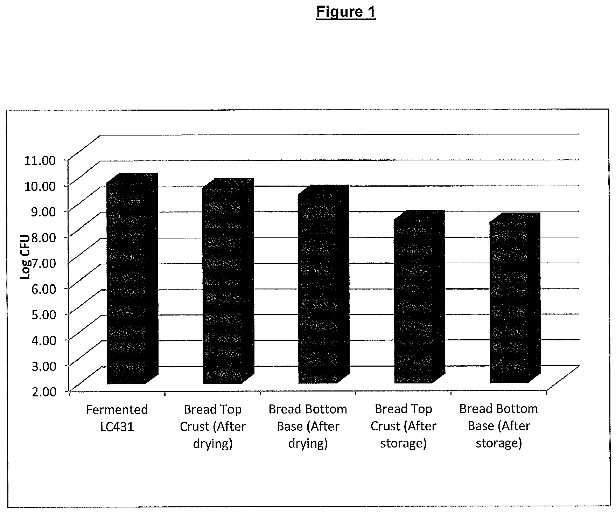

As can be seen in Table 1 below (and subsequently in Figure 1), the Log CFU, the process of applying and then drying the composition on the top crust and bottom base of the bread resulted in very little cell viability loss. After storage, minor losses in cell viability were recorded in both samples, but were still well above the commercially and therapeutic level of 7.0 Log CFU required. It should be noted that various types of breads were tested using this method with similar probiotic viability results observed. The water activity of the breads before application of probiotics generally ranged between 0.75 to 0.94, and after drying, water activity ranged from 0.66 to 0.74.

**Table 1: Analysis of Log CFU**

| Sample | Trial 1 | Trial 2 | Trial 3 | Avg |
|---|---|---|---|---|
| Fermented LC431 (control) | 9.98 | 9.7 | 9.82 | 9.83 |
| Bread top crust (after drying) | 9.7 | 9.65 | 9.5 | 9.62 |
| Bread bottom base (after drying) | 9.43 | 9.3 | 9.32 | 9.35 |
| Bread top crust (after storage) | 8.42 | 8.26 | 8.38 | 8.35 |
| Bread bottom base (after storage) | 8.3 | 8.22 | 8.21 | 8.24 |

### Example 5: Analysis of Log CFU following preparation and storage of fortified LC431, B. lactis 12 (BB12) and L. acidophilus 05 (LA05) combination probiotic bread.

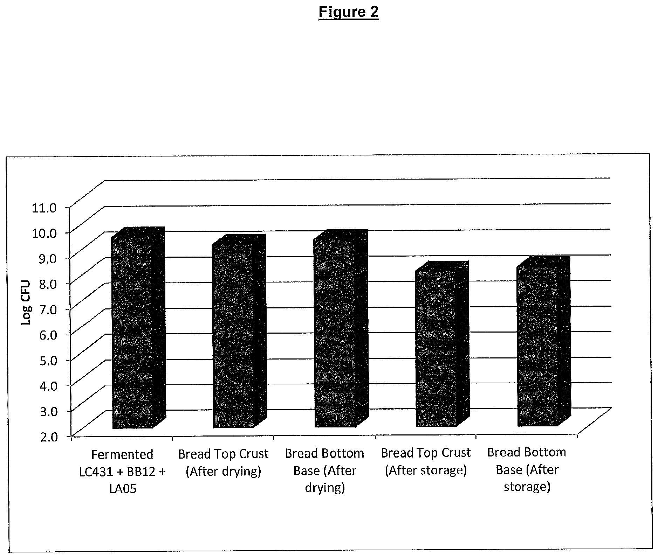

A probiotic LC431, BB12 and LA05 combination fortified bread was prepared according to Example 3, although using all three micro-organisms as the inoculant, not just LC431. The results are shown in Table 2 (and also Figure 2).

Similar to Example 4, the inventors then tested the viability of the fortified combination probiotic cells. Similar beneficial and surprising results were seen in this trial showing that the inventive concept is not limited to specific probiotic types and may similarly also include combinations of probiotics.

**Table 2: Analysis of Log CFU**

| Sample | Trial 1 | Trial 2 | Avg |
|---|---|---|---|
| Fermented LC431 + BB12 + LA05 | 9.42 | 9.6 | 9.5 |
| Bread top crust (after drying) | 9.1 | 9.3 | 9.2 |
| Bread bottom base (after drying) | 9.3 | 9.45 | 9.4 |
| Bread top crust (after storage) | 8.02 | 8.23 | 8.1 |
| Bread bottom base (after storage) | 8.3 | 8.25 | 8.3 |

### Example 6: Analysis of Log CFU following preparation and storage of fortified Lactobacillus plantarum 299V.

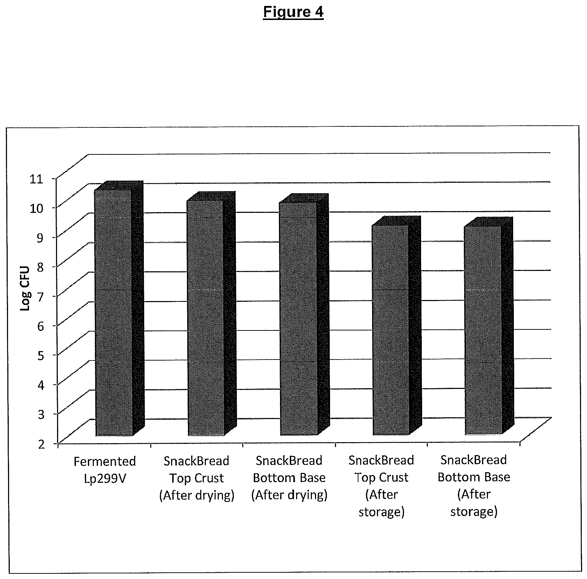

A similar study was performed to Example 5, but this time using *Lactobacillus plantarum* 299V. As discussed previously, this probiotic micro-organism is an attractive target owing to its usefulness in treating IBS. The results are shown in Figure 4.

The absolute values after five days were 9.12 and 9.07 log CFU per bread (top and bottom crust after storage, respectively), which translates into 1.3 billion and 1.17 billion of viable cells delivered per bread. This is an important commercial achievement, because the Applicant has been able to apply the invention using a wide range of different probiotic organisms, and in the case of *Lactobacillus plantarum* 299V, the results are showing extremely good probiotic cell viability counts after a five day storage trial.

Therefore the delivery of the clinically validated and popular probiotic strain *Lactobacillus plantarum* 299v via fortified breads may be a very attractive option to the targeted consumers suffering from a common gastro-intestinal disorder called Irritable Bowel Syndrome.

### Example 7: Comparison of pre-fermented composition freshly applied to bread to commercially available probiotic drinks.

To test the stability of different probiotic strains and the benefit of pre-fermenting the microbes in the composition, a test composition with LC431 (fermented in 8.0% (w/w) reconstituted skim milk and 4.0% (w/w) dextrose monohydrate as described previously) was compared to two commercially available probiotic drinks (Yakult and Activate).

For each group, 1 ml of the composition was spread on each piece of bread and dried at 50°C for 15 min in a convection type forced air oven. A micro-pipette was used to distribute 1 ml of the liquid in mini droplet forms over the bread surfaces and spread evenly with the help of a spatula. The samples were then dried at 50°C for 15 min in a convection type forced air oven. The bread samples were then packed in LDPE sachets and stored in the laboratory incubator maintained at a constant temperature of 25°C for 5 days. Probiotic viability was tested before and after application to the top and bottom crust of the bread's surfaces, and also after the storage for 5 days.

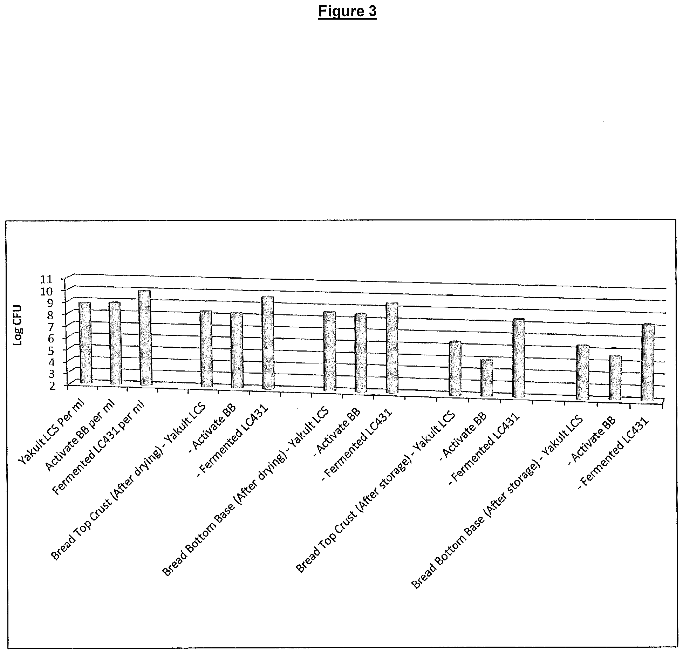

The results are shown in Figure 3. The fermented curd containing single strain of L. *casei* 431 yielded very high concentrations of viable cells to the tune of 9.9 log CFU. The drying losses in viability were minimal and recorded as 9.7 and 9.43 respectively for bread tops and bottoms. Moreover, only 1.3 and 1.1 log cell reductions were observed on bread top and bottom respectively, after the storage period. This was compared to the commercially available products which lost cell viability much more rapidly. For the test composition, the absolute values after 5 days were 8.4 and 8.3 log CFU, which translates into 250 million and 200 million of viable cells delivered per bread. These were more than 10 folds higher than the benchmark of delivering at least 10 million cells per bread.

### Example 8: Sensory analysis of Probiotic Bread

A sensory trial was performed to determine the acceptability of the taste, feel and appearance of a fortified probiotic bread sample 1 (as used in Example 4) and a probiotic bread sample 2 (as used in Example 5) compared to the control (normal, non probiotic bread). Scores were provided for acceptability from a scale starting at 1 (poor acceptability) to 9 (excellent acceptability).

As can be seen in Table 3 below, the probiotic bread samples 1 and 2 are very comparable in terms of each specific test, and overall acceptability, to the control bread sample.

**Table 3: Sensory analysis**

| Test | Control | Probiotic Bread Sample 1 | Probiotic Bread Sample 2 |
|---|---|---|---|
| Crust appearance | 6 | 6 | 6.5 |
| Odour | 7.5 | 7 | 6.7 |
| Crust colour | 6.5 | 6.85 | 6.55 |
| Crispiness | 7 | 7.2 | 6.8 |
| Crust hardness | 8 | 7 | 6.7 |
| Taste | 7.6 | 7.2 | 7.8 |
| Overall acceptability | 7.4 | 7.1 | 6.9 |

### Example 9: Moisture analysis of probiotic bread

The crust moisture content (%) and water activity of the probiotic bread samples 1 and 2 are only slightly higher than the control. These values are acceptable for commercial purposes, and it is unlikely that consumers will be able to notice any significant differences, as exemplified by the acceptability analysis shown in Example 6.

The slightly higher moisture content in the test samples will not adversely affect the shelf life of the bread, and it is still expected that the probiotic fortified bread will retain the full five day shelf life requirements.

**Table 4: Moisture analysis**

| Test | Control | Probiotic Bread Sample 1 | Probiotic Bread Sample 2 |
|---|---|---|---|
| Crust moisture content % | 7.2 | 8.1 | 7.85 |
| Crust water activity | 0.75 | 0.74 | 0.72 |

## Claims

1. A method of manufacturing a probiotic fortified food product, optionally bread, bread snack or muesli bar, including
a. pre-inoculating a composition that comprises an amount of milk, milk solids, or reconstituted milk with an amount of the single species of probiotic micro-organism or combination of different probiotic micro-organisms, wherein other than the probiotic micro-organism, the composition only includes components which are either fully milk-derived or are inherently found in milk;
b. incubating the composition during an early stationary growth phase of the probiotic micro-organism(s) until a probiotic culture in the composition is achieved with a cell count above 7 log CFU ml⁻¹;
c. applying the composition including the at least one probiotic organism to at least one portion of a surface of the food product.

2. The method of claim 1 wherein following step b. and before step c., the composition is homogenised to form a liquid with a viscosity below 4 cP, for example between 1 to 3 cP.

3. The method as claimed in claim 1 wherein, at step b., the composition is inoculated with the at least one micro-organism to achieve a cell count above 9.0 log CFU ml⁻¹

4. The method according to claim 1 wherein the food product has a water activity (a_{w}) level between 0.5 to 0.9.

5. The method according to claim 1 wherein at least one species of probiotic micro-organism is selected from the group consisting of a lactic acid bacteria, non-lactic acid bacteria and non-pathogenic yeast, for example wherein at least one probiotic micro-organism is selected from the group consisting of *Lactobacillus acidophilus, Lactobacillus bulgaricus, Lactobacillus casei, Bifidobacterium* (*Lactobacillus bifidus*), *Streptococcus thermophilus,* and combinations thereof.

6. The method according to claim 1 wherein the composition includes reconstituted milk and/or at least one coagulated component, for example, wherein the at least one coagulated component is protein and a coagulant is lactic acid.

7. The method according to claim 1 wherein the composition includes a milk-derived sugar, for example selected from the group consisting of lactose, glucose and/or galactose.

8. The method according to claim 1 wherein prior to step a., the composition is supplemented with additional amounts of at least one sugar selected from a type of sugar inherently present in milk including lactose, glucose and/or galactose.

9. The method according to claim 1 wherein prior to step a., the composition is configured to include a sugar concentration between 3 to 20 % (w/v), for example between 7-10 % (w/v).

10. The method according to claim 1 or 9, wherein prior to step a., the composition is configured to include less than 5% fat (w/v), for example substantially no fat.

11. The method according to claim 1 wherein at step c., the composition is spread as the thin layer less than 0.1 mm thick, for example less than 0.05 mm thick.

12. The method according to claim 1 or claim 11, wherein at step c., the method includes applying between 200µl to 2 ml , for example between 500 µl to 1 ml, of the composition to the surface of the food product.

13. The method according to claim 1 or claim 11, wherein at step c., the method includes spreading the composition across an entire surface of the food product.

14. The method according to claim 1 or claim 11, wherein at step c., the method includes spraying the composition on an entire surface of the food product.

15. The method according to claim 1, wherein following step c., the method includes the further step of
d. drying the composition on the surface of the food product at a temperature below 70°C or at a temperature between 30°C to 70°C, optionally between 50°C to 60°C, for example for between 10 to 120 minutes and/or until the water activity (a_{w}) of the composition applied to the surface of the food product and/or surfaces of the food product is between 0.5 to 0.9, and/or closely matches the original water activity (a_{w}) of the food product before applying the composition to its surface.

## Patentansprüche

1. Verfahren zur Herstellung eines probiotischen angereicherten Lebensmittelprodukts, optional Brot, Brotsnack oder Müsliriegel, umfassend
a. Vorinokulieren einer Zusammensetzung, die eine Menge an Milch, Milchfeststoffen oder rekonstituierter Milch umfasst, mit einer Menge der einzelnen Spezies eines probiotischen Mikroorganismus oder einer Kombination von verschiedenen probiotischen Mikroorganismen, wobei die Zusammensetzung außer dem probiotischen Mikroorganismus ausschließlich Komponenten enthält, die entweder vollständig von Milch abgeleitet sind oder inhärent in Milch gefunden werden,
b. Inkubieren der Zusammensetzung während einer frühen stationären Wachstumsphase des probiotischen Mikroorganismus oder der probiotischen Mikroorganismen, bis eine probiotische Kultur in der Zusammensetzung erreicht ist mit einer Zellzahl oberhalb von 7 log KBE ml⁻¹,
c. Aufbringen der wenigstens einen probiotischen Mikroorganismus umfassenden Zusammensetzung auf wenigstens einen Bereich einer Oberfläche des Lebensmittelprodukts.

2. Verfahren nach Anspruch 1, worin im Anschluss an Schritt b. und vor Schritt c. die Zusammensetzung homogenisiert wird, um eine Flüssigkeit mit einer Viskosität unter 4 cP, zum Beispiel zwischen 1 und 3 cP, zu ergeben.

3. Verfahren nach Anspruch 1, worin in Schritt b. die Zusammensetzung mit dem wenigstens einen Mikroorganismus inokuliert wird, um eine Zellzahl oberhalb von 9.0 log KBE ml⁻¹ zu ergeben.

4. Verfahren nach Anspruch 1, worin das Lebensmittelprodukt ein Wasseraktivitätslevel (a_{w}) zwischen 0,5 und 0,9 besitzt.

5. Verfahren nach Anspruch 1, worin die wenigstens eine Spezies eines probiotischen Mikroorganismus ausgewählt ist aus der Gruppe, bestehend aus Milchsäurebakterien, Nicht-Milchsäurebakterien und nicht-pathogener Hefe, zum Beispiel, worin wenigstens ein probiotischer Mikroorganismus ausgewählt ist aus der Gruppe, bestehend aus *Lactobacillus acidophilus, Lactobacillus bulgaricus, Lactobacillus casei, Bifidobacterium (Lactobacillus bifidus), Streptococcus thermophilus,* und Kombinationen davon.

6. Verfahren nach Anspruch 1, worin die Zusammensetzung rekonstituierte Milch und/oder wenigstens eine koagulierte Komponente umfasst, zum Beispiel, worin die wenigstens eine koagulierte Komponente ein Protein ist und ein Koagulans Milchsäure ist.

7. Verfahren nach Anspruch 1, worin die Zusammensetzung einen von Milch abgeleiteten Zucker, zum Beispiel ausgewählt aus der Gruppe, bestehend aus Lactose, Glucose und/oder Galactose, umfasst.

8. Verfahren nach Anspruch 1, worin vor Schritt a. die Zusammensetzung mit einer zusätzlichen Menge wenigstens eines Zuckers versehen wird, der ausgewählt ist aus einem inhärent in Milch vorhandenen Zuckertypen, einschließlich Lactose, Glucose und/oder Galactose.

9. Verfahren nach Anspruch 1, worin vor Schritt a. die Zusammensetzung konfiguriert wird, eine Zuckerkonzentration zwischen 3 und 20 % (w/v), zum Beispiel zwischen 7 und 10 % (w/v), zu enthalten.

10. Verfahren nach Anspruch 1 oder 9, worin vor Schritt a. die Zusammensetzung konfiguriert wird, weniger als 5 % (w/v) Fett, zum Beispiel im Wesentlichen kein Fett, zu enthalten.

11. Verfahren nach Anspruch 1, worin in Schritt c. die Zusammensetzung als dünne, weniger als 0,1 mm dicke Schicht, zum Beispiel weniger als 0,05 mm dicke Schicht, aufgetragen wird.

12. Verfahren nach Anspruch 1 oder Anspruch 11, worin in Schritt c. das Verfahren das Auftragen von zwischen 200 µl und 2 ml, zum Beispiel von zwischen 500 µl und 1 ml, der Zusammensetzung auf die Oberfläche des Lebensmittelprodukts umfasst.

13. Verfahren nach Anspruch 1 oder Anspruch 11, worin in Schritt c. das Verfahren das Verteilen der Zusammensetzung über die gesamte Oberfläche des Lebensmittelprodukts umfasst.

14. Verfahren gemäß Anspruch 1 oder Anspruch 11, worin in Schritt c. das Verfahren das Sprühen der Zusammensetzung auf die gesamte Oberfläche des Lebensmittelprodukts umfasst.

15. Verfahren gemäß Anspruch 1, worin im Anschluss an Schritt c. das Verfahren den weiteren Schritt umfasst:
d. Trocknen der Zusammensetzung auf der Oberfläche des Lebensmittelprodukts bei einer Temperatur unter 70 °C oder einer Temperatur zwischen 30 °C und 70 °C, optional zwischen 50 °C und 60 °C, zum Beispiel für 10 bis 120 Minuten und/oder, bis die Wasseraktivität (a_{w}) der Zusammensetzung, die auf die Oberfläche des Lebensmittelprodukts und/oder Oberflächen des Lebensmittelprodukts aufgetragen ist, zwischen 0,5 und 0,9 liegt und/oder der ursprünglichen Wasseraktivität (a_{w}) des Lebensmittelprodukts vor dem Auftragen der Zusammensetzung auf seine Oberfläche nahezu entspricht.

## Revendications

1. Procédé de fabrication d'un produit alimentaire enrichi en probiotiques, éventuellement du pain, un en-cas à base de pain ou une barre de muesli, comprenant
a. la pré-inoculation d'une composition qui comprend une certaine quantité de lait, de solides de lait ou de lait reconstitué avec une certaine quantité de l'espèce seule de microorganisme probiotique ou d'une combinaison de différents microorganismes probiotiques, ladite composition comprenant, à part ledit microorganisme probiotique, uniquement des composants soit qui sont intégralement dérivés de lait soit qui se trouvent de façon inhérente dans le lait ;
b. l'incubation de la composition pendant une phase de croissance stationnaire précoce du ou des microorganismes probiotiques jusqu'à ce qu'une culture probiotique dans la composition soit obtenue avec une numération cellulaire supérieure à 7 log UFC ml⁻¹ ;
c. l'application de la composition comprenant ledit au moins un organisme probiotique sur au moins une partie d'une surface du produit alimentaire.

2. Procédé selon l'étape 1, dans lequel suite à l'étape b. et avant l'étape c., la composition est homogénéisée pour former un liquide présentant une viscosité inférieure à 4 cP, par exemple comprise entre 1 à 3 cP.

3. Procédé selon l'étape 1, dans lequel, à l'étape b., la composition est inoculée avec ledit au moins un microorganisme pour atteindre une numération cellulaire supérieure à 9,0 log UFC ml⁻¹.

4. Procédé selon la revendication 1, dans lequel le produit alimentaire présente un niveau d'activité hydrique (a_{w}) compris entre 0,5 à 0,9.

5. Procédé selon la revendication 1, dans lequel au moins une espèce de microorganisme probiotique est choisie dans le groupe constitué par une bactérie d'acide lactique, une bactérie non d'acide lactique et une levure non pathogène, par exemple ledit au moins un microorganisme probiotique étant choisi dans le groupe constitué par *Lactobacillus acidophilus, Lactobacillus bulgaricus, Lactobacillus casei, Bifidobacterium (Lactobacillus bifidus), Streptococcus thermophilus,* et des combinaisons de ceux-ci.

6. Procédé selon la revendication 1, dans lequel la composition comprend du lait reconstitué et/ou au moins un composant coagulé, par exemple, ledit au moins un composant coagulé étant une protéine et un coagulant étant l'acide lactique.

7. Procédé selon la revendication 1, dans lequel la composition comprend un sucre dérivé de lait, par exemple choisi dans le groupe constitué par le lactose, le glucose et/ou le galactose.

8. Procédé selon la revendication 1, dans lequel avant l'étape a., la composition est complétée par des quantités supplémentaires d'au moins un sucre choisi parmi un type de sucre présent de manière inhérente dans le lait dont le lactose, le glucose et/ou le galactose.

9. Procédé selon la revendication 1, dans lequel avant l'étape a., la composition est conçue pour comprendre une concentration de sucre comprise entre 3 à 20 % (p/v), par exemple entre 7 à 10 % (p/v).

10. Procédé selon la revendication 1 ou 9, dans lequel avant l'étape a., la composition est conçue pour comprendre moins de 5 % de graisses (p/v), par exemple sensiblement aucune graisse.

11. Procédé selon la revendication 1, dans lequel à l'étape c., la composition est étalée sous la forme d'une couche mince faisant moins de 0,1 mm d'épaisseur, par exemple moins de 0,05 mm d'épaisseur.

12. Procédé selon la revendication 1 ou 11, dans lequel à l'étape c., le procédé comprend l'application d'entre 200 µl à 2 ml, par exemple d'entre 500 µl à 1 ml, de la composition à la surface du produit alimentaire.

13. Procédé selon la revendication 1 ou 11, dans lequel à l'étape c., le procédé comprend l'étalement de la composition sur une surface entière du produit alimentaire.

14. Procédé selon la revendication 1 ou 11, dans lequel à l'étape c., le procédé comprend la pulvérisation de la composition sur une surface entière du produit alimentaire.

15. Procédé selon la revendication 1, dans lequel suite à l'étape c., le procédé comprend l'étape supplémentaire de
d. séchage de la composition sur la surface du produit alimentaire à une température inférieure à 70°C ou à une température comprise entre 30°C à 70°C, éventuellement entre 50°C à 60°C, par exemple pendant entre 10 à 120 minutes et/ou jusqu'à ce que l'activité hydrique (a_{w}) de la composition appliquée sur la surface du produit alimentaire et/ou des surfaces du produit alimentaire soit comprise entre 0,5 à 0,9, et/ou correspond étroitement à l'activité hydrique originale (a_{w}) du produit alimentaire avant application de la composition à sa surface.
